(19)

Europäisches
Patentamt
European
Patent Office
Office européen
des brevets

(11) **EP 3 130 290 A1**

(12) **EUROPEAN PATENT APPLICATION**
published in accordance with Art. 153(4) EPC

(43) Date of publication:
**15.02.2017 Bulletin 2017/07**

(51) Int Cl.:
***A61B 8/06*** (2006.01)      ***A61B 8/14*** (2006.01)

(21) Application number: **15776286.5**

(86) International application number:
**PCT/JP2015/057347**

(22) Date of filing: **12.03.2015**

(87) International publication number:
**WO 2015/156081 (15.10.2015 Gazette 2015/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **08.04.2014 JP 2014079533**

(71) Applicant: **Hitachi, Ltd.
Chiyoda-ku,
Tokyo 100-8280 (JP)**

(72) Inventor: **SEKI, Yoshinori
Mitaka-shi
Tokyo 181-8622 (JP)**

(74) Representative: **Strehl Schübel-Hopf & Partner
Maximilianstrasse 54
80538 München (DE)**

(54) **ULTRASONIC DIAGNOSING DEVICE**

(57)    The purpose of the invention is to find each component of a blood flow velocity using a simple process. In a secondary beam method, scanning is performed using Doppler measurement ultrasound beams (42) as primary beams, and Doppler measurement components are measured on the basis of the ultrasound waves received from the direction of each Doppler measurement ultrasound beam. Then, ultrasound waves forming secondary beams (58) at a direction that intersects the direction of the Doppler measurement ultrasound beams are sent and received, and the Doppler effect for the secondary beam direction component at the point of intersection of the Doppler measurement ultrasound beam (42) with the secondary beams (58) is measured. Furthermore, using the intersection point as the position for starting integration, an integration calculation based on the law of conservation of mass is performed along the route that intersects with the Doppler measurement ultrasound beams (42), and the component in the direction of the intersection route is found. The initial value for integration is the component in the direction of the intersection route of the integration start point (PA), and is found on the basis of the Doppler measurement component and the secondary beam direction component at the integration start point.

FIG. 5

## Description

### TECHNICAL FIELD

The present disclosure relates to an ultrasound diagnostic apparatus, and in particular to an apparatus which measures a bloodstream velocity.

### BACKGROUND

Ultrasound diagnostic apparatuses which measure a bloodstream velocity of a target by a Doppler method are in wide use. In such ultrasound diagnostic apparatuses, a bloodstream velocity which is a vector quantity is displayed over a tomographic image in an overlapping manner with an arrow or the like, so as to enable diagnosis of circulatory organs such as a blood vessel and a heart. In an ultrasound diagnostic apparatus which executes such a VFM (Vector Flow Mapping), the bloodstream velocity is measured using the Doppler method. In the Doppler method, of the components of the bloodstream velocity, only the component in a direction of transmission/reception of the ultrasound is measured, and thus, it is difficult to measure a component in a direction orthogonal to the transmission/reception direction of the ultrasound. In consideration of this, as techniques for determining two components of the bloodstream velocity, techniques described in Patent Documents 1 and 2 are known.

Patent Document 1 discloses a technique in which a component of the bloodstream velocity in a direction of the ultrasound beam is measured with the Doppler method, and a component in a direction orthogonal to the ultrasound beam (orthogonal direction component) is determined through a calculation. FIG. 15 conceptually shows a velocity detection process described in Patent Document 1. In this process, an orthogonal direction component $V_\theta$ of the bloodstream velocity is determined from an equation of continuity. First, for each ultrasound beam direction, the ultrasound beam direction component $V_r$ of the bloodstream velocity is measured using the Doppler method. Then, in an orthogonal path C orthogonal to the ultrasound beam, an amount of change toward the orthogonal path direction of the orthogonal direction component $V_\theta$ is determined using the beam direction component $V_r$, and further, the amount of change is integrated along the orthogonal path C to determine the orthogonal direction component $V_\theta$. An integration start position P is a position on a wall surface W of the circulatory organ such as the heart, the blood vessel, or the like, and an initial value of the integration is an orthogonal path direction component Vw of a motion velocity of the integration start position P.

Patent Document 2 discloses an ultrasound diagnostic apparatus in which scanning of ultrasound beam is executed for each of two scanning conditions having the scanning planes of the ultrasound beam shifted from each other, and the bloodstream velocity is determined based on each ultrasound beam direction component which is Doppler-measured for each scanning condition. In this ultrasound diagnostic apparatus, based on an ultrasound beam direction component measured with a first scanning condition and an ultrasound beam direction component measured with a second scanning condition, the bloodstream velocity at a common orthogonal coordinate is determined. Because the scanning planes are shifted from each other between the two scanning conditions, the direction of the ultrasound beam due to the first scanning condition and the direction of the ultrasound beam due to the second scanning condition have different directions at each point in the orthogonal coordinate. With this process, a vector calculation is executed based on the ultrasound beam direction components, and each axial direction component of the bloodstream velocity is determined.

### CITATION LIST

### PATENT LITERATURE

**[0005]**

　　Patent Document 1: JP 2013-192643 A
　　Patent Document 2: JP 2013-165922 A

### SUMMARY

### TECHNICAL PROBLEM

In the ultrasound diagnostic apparatus described in Patent Document 1, the integration start position P on the orthogonal path C is set at a position on the wall surface W of the circulatory organ, and the initial value of the integration is set at the orthogonal path direction component Vw of the motion velocity of the integration start position P. The integration start position P is determined based on a tomographic image, and the initial value of the integration is determined based on a plurality of tomographic images sequentially acquired with elapse of time. More specifically, based on pattern matching of a plurality of tomographic image data acquired with elapse of time, a pattern of the wall surface of the circulatory organ is tracked, and the initial value of the integration is determined by determining the motion velocity of each integration start position acquired for the plurality of tomographic image data. However, depending on the shape of the circulatory organ and the measurement state, there may be cases where the integration start position P cannot be acquired and the orthogonal direction component of the bloodstream velocity cannot be determined.

In the ultrasound diagnostic apparatus described in Patent Document 2, in all regions where each component of the bloodstream velocity is determined, two ultrasound beam scans with two scanning conditions are executed. Therefore, the load of measurement process may become heavy.

One advantage of the present disclosure lies in determination of components of the bloodstream velocity with a simple process.

SOLUTION TO PROBLEM

According to one aspect of the present disclosure, there is provided an ultrasound diagnostic apparatus comprising: a transmission and reception portion that transmits and receives ultrasound; a main beam controller that controls the transmission and reception portion, to scan a main beam formed by ultrasound transmitted and received by the transmission and reception portion; a main Doppler measurement portion that Doppler-measures a main beam direction component of a bloodstream velocity for each main beam direction based on ultrasound received by the transmission and reception portion from each main beam direction; a calculator that determines an intersecting path direction component of the bloodstream velocity for a positon on an intersecting path that intersects each main beam direction based on each main beam direction component of the bloodstream velocity on the intersecting path; a sub-beam controller that causes the transmission and reception portion to transmit and receive ultrasound for forming a sub-beam passing through a point on the intersecting path; and a sub-Doppler measurement portion that Doppler-measures a sub-beam direction component of the bloodstream velocity at a passing point of the sub-beam on the intersecting path based on ultrasound received by the transmission and reception portion from the sub-beam direction, wherein the sub-beam has a direction different from a direction of the main beam passing through the passing point, and the calculator determines the intersecting path direction component of the bloodstream velocity using the sub-beam direction component of the bloodstream velocity at the passing point.

In the present disclosure, a main beam formed by the ultrasound which is transmitted and received is scanned, and the main beam direction component of the bloodstream velocity is Doppler-measured based on the ultrasound received from each main beam direction. In general, in the Doppler measurement, it is difficult to determine the bloodstream velocity component in a direction intersecting the ultrasound beam. In consideration of this, in the present disclosure, based on each main beam direction component of the bloodstream velocity on the intersecting path, the intersecting path direction component of the bloodstream velocity is determined for the position on the intersecting path. The bloodstream velocity is determined as a combination of the main beam direction component and the intersecting path direction component determined in this manner. When the intersecting path direction component of the bloodstream velocity is determined, the sub-beam direction component at a passing point of the sub-beam on the intersecting path is Doppler-measured, and the sub-beam direction component is used. Alternatively, the sub-beam may be a beam which intersects the main beam at one passing point on the intersecting path.

According to another aspect of the present disclosure, the calculator determines a main beam direction change amount which is an amount of change of each main beam direction component of the bloodstream velocity on the intersecting path in the respective main beam direction, and executes an integration calculation to integrate each main beam direction change amount along the intersecting path, and the calculator determines an initial condition of the integration calculation based on an intersecting path direction component of the sub-beam direction component of the bloodstream velocity at the passing point.

According to another aspect of the present disclosure, the passing point is a point at one end of the intersecting path, and the calculator sets a position of the one end as the initial condition of the integration calculation and executes the integration calculation along the intersecting path.

In the present disclosure, the amount of change of the main beam direction on the intersecting path is integrated along the intersecting path. With this process, integration based on the law of conservation of mass is executed, and the path direction component of the bloodstream velocity is determined. The integration based on the law of conservation of mass is based on an equation of continuity indicating that a flow amount of blood flowing into a certain infinitesimal region and a flow amount of the blood flowing out of the same infinitesimal region are equal to each other. The initial condition of the integration based on the law of conservation of mass is determined, for example, from a plurality of tomographic image data acquired with the elapse of time. However, depending on the shape of the circulatory organ and the measurement state, it may be difficult to acquire the initial condition from the plurality of tomographic image data. In the present disclosure, the initial condition of the integration calculation is determined based on the sub-beam direction component at the passing point of the sub-beam on the intersecting path. With this process, the initial condition of the integration calculation can be acquired without the use of the tomographic image data.

According to another aspect of the present disclosure, the passing point is a partway point on the intersecting path, and the calculator comprises: a first integrator that sets a position of the partway point as the initial condition and executes the integration calculation along the intersecting path from the partway point on one side; a second integrator that sets the position of the partway point as the initial condition and executes the integration calculation along the intersecting path from the partway point on the other side; and a combiner that determines the intersecting path direction component of the bloodstream velocity based on calculation results by the first integrator and the second integrator.

According to another aspect of the present disclosure, the sub-beam controller causes the transmission and reception portion to transmit and receive ultrasound that forms, as the sub-beams, a first sub-beam having one end of the intersecting path as the passing point and a second sub-beam having the other end of the intersecting path as the passing point, and the calculator comprises: a first integrator that sets a position of the one end as the initial condition of the integration calculation, and executes the integration calculation along the intersecting path; a second integrator that sets a position of the other end as the initial condition of the integration calculation, and executes the integration calculation along the intersecting path; and a combiner that determines the intersecting path direction component of the bloodstream velocity based on calculation results by the first integrator and the second integrator.

According to another aspect of the present disclosure, the ultrasound diagnostic apparatus further comprises: a B-mode controller that controls the transmission and reception portion to scan a B-mode beam formed by the ultrasound transmitted and received by the transmission and reception portion; a tomographic image producer that produces tomographic image data based on ultrasound received by the transmission and reception portion from each B-mode beam direction; and a velocity calculator that determines the intersecting path direction component of the bloodstream velocity at one end of the intersecting path based on a plurality of tomographic image data produced with elapse of time, wherein the calculator comprises a first integrator that sets, as the initial conditions, an intersecting path direction component of the bloodstream velocity at the one end and a position of the one end, and executes the integration calculation along the intersecting path, the passing point is a point at the other end of the intersecting path, and the calculator further comprises: a second integrator that sets a position of the other end as the initial condition of the integration calculation, and executes the integration calculation along the intersecting path; and a combiner that determines the intersecting path direction component of the bloodstream velocity based on calculation results by the first integrator and the second integrator.

In the present disclosure, the intersecting path direction component of the bloodstream velocity is determined based on the calculation results of the first integrator and the second integrator. With this configuration, two integration results are reflected in the bloodstream velocity, and the reliability of the determined bloodstream velocity can be improved as compared to a configuration with one integration result.

According to another aspect of the present disclosure, the sub-beam controller causes the transmission and reception portion to transmit and receive ultrasound for forming an additional sub-beam passing through the passing point, the sub-Doppler measurement portion determines an additional sub-beam direction component of the bloodstream velocity for the passing point based on ultrasound received by the transmission and reception portion from the additional sub-beam direction, the additional sub-beam has a direction different from each of the directions of the main beam and the sub-beam passing through the passing point, and the calculator determines the intersecting path direction component of the bloodstream velocity at the passing point using the sub-beam direction component and the additional sub-beam direction component of the bloodstream velocity.

In the present disclosure, the intersecting path direction component of the bloodstream velocity at the passing point of the sub-beam on the intersecting path is determined using the additional sub-beam direction component in addition to the sub-beam direction component of the bloodstream velocity. With such a configuration, the measurement results by a plurality of sub-beams are reflected in the bloodstream velocity, and the reliability of the determined bloodstream velocity can be improved as compared to a configuration with one sub-beam.

ADVANTAGEOUS EFFECTS OF INVENTION

According to the present disclosure, the components of the bloodstream velocity can be determined with a simple process.

BRIEF DESCRIPTION OF DRAWINGS

FIG. 1 is a diagram showing a structure of an ultrasound diagnostic apparatus.
FIG. 2 is a diagram conceptually showing a relationship between a tomographic image and a Doppler measurement component.

FIG. 3 is a diagram conceptually showing a process at an integration based on the law of conservation of mass.

FIG. 4 is a diagram showing an incomplete region.

FIG. 5 is a diagram explaining a sub-beam method.

FIG. 6 is a diagram showing a relationship between vectors.

FIG. 7 is a diagram explaining a two-end beam method.

FIG. 8 is a diagram explaining a single sub-beam method.

FIG. 9 is a diagram showing an example configuration of determining a bloodstream velocity of a vascular lumen with only a sub-beam method.

FIG. 10 is a diagram showing an example configuration of determining a bloodstream velocity of a vascular lumen by combining a sub-beam method and a wall-surface method.

FIG. 11 is a diagram showing an example configuration of determining two $\beta$-axis direction components in each region.

FIG. 12 is a diagram showing an example configuration of determining two $\beta$-axis direction components in each region.

FIG. 13 is a diagram conceptually showing a tomographic image and an ultrasound beam for Doppler measurement by a sector scanning.

FIG. 14 is an enlarged view of a region near an incomplete region.

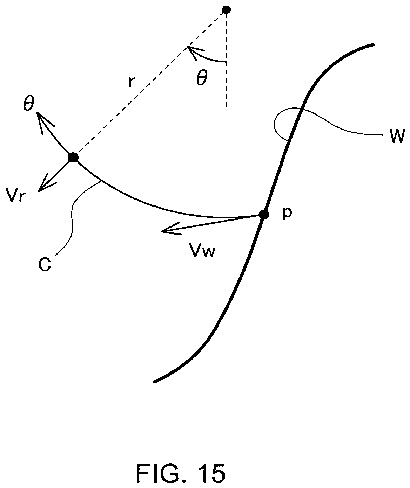

FIG. 15 is a diagram conceptually showing a velocity detection process in the related art.

## DESCRIPTION OF EMBODIMENTS

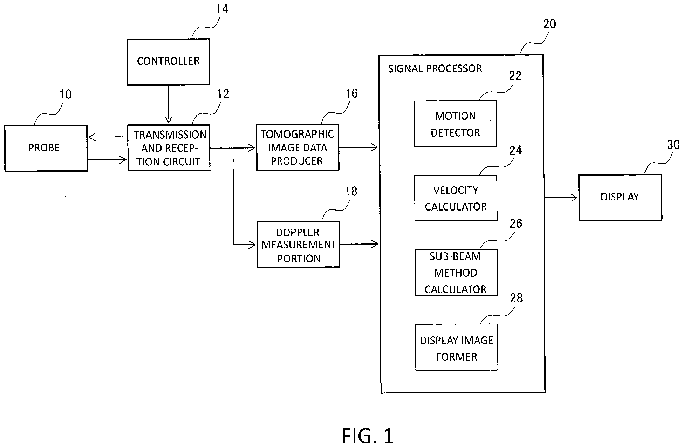

FIG. 1 shows an ultrasound diagnostic apparatus according to an embodiment of the present disclosure. The ultrasound diagnostic apparatus scans an ultrasound beam which is transmitted and received to and from a target, displays a tomographic image based on the received ultrasound, and measures and displays a bloodstream velocity in the circulatory organ of the target. The bloodstream velocity is a vector quantity having a direction and a magnitude, and is displayed by a figure such as an arrow or the like, a combination of colors and brightness, or two component values.

In measurement, a probe 10 is set in a state of contacting a surface of the target. The probe 10 has a plurality of ultrasound transducers. A transmission and reception circuit 12 transmits a transmission signal to each ultrasound transducer of the probe 10 based on control by a controller 14. With this process, ultrasound is transmitted from the probe 10. When ultrasound reflected in the target is received by each ultrasound transducer of the probe 10, each ultrasound transducer outputs an electric signal to the transmission and reception circuit 12. The transmission and reception circuit 12 applies level adjustment or the like on the electric signal which is output from each ultrasound transducer, and applies phasing addition.

The ultrasound diagnostic apparatus displays the tomographic image by a B-mode measurement as will be described below. According to the control by the controller 14, the transmission and reception circuit 12 forms a transmission ultrasound beam at the probe 10, and scans the transmission ultrasound beam toward the target. In addition, according to the control by the controller 14, the transmission and reception circuit 12 phase-adds the electric signal which is output from the ultrasound transducers of the probe 10, to produce a reception signal for B-mode measurement, and outputs the produced signal to a tomographic image data producer 16. With this process, a reception ultrasound beam is formed at the probe 10, and a reception signal corresponding to the reception ultrasound beam is output as a reception signal for B-mode measurement from the transmission and reception circuit 12 to the tomographic image data producer 16.

The tomographic image data producer 16 produces tomographic image data based on the reception signal acquired with respect to each ultrasound beam direction, and outputs the produced data to a signal processor 20. The signal processor 20 displays on a display 30 a tomographic image based on the tomographic image data.

The ultrasound diagnostic apparatus determines the bloodstream velocity by a Doppler measurement as described below, and displays on the display 30 the bloodstream velocity in an overlapping manner on the tomographic image. The transmission and reception of the ultrasound for B-mode measurement and the transmission and reception of the ultrasound for Doppler measurement are executed in a time-divisional manner, and the B-mode measurement and the Doppler measurement are executed in a time-divisional manner.

The controller 14 controls the transmission and reception circuit 12 to scan the transmission ultrasound beam formed at the probe 10, and transmits ultrasound for Doppler measurement in each transmission ultrasound beam direction. A region in which the ultrasound beam for Doppler measurement is scanned is within a region in which the ultrasound beam for B-mode measurement is scanned. The transmission and reception circuit 12 phase-adds the electric signal which is output from the ultrasound transducers of the probe 10 according to the control by the controller 14, to produce a reception signal for Doppler measurement, and outputs the produced signal to a Doppler measurement portion 18. With this process, a reception ultrasound beam is formed at the probe 10, and a reception signal corresponding to the reception ultrasound beam is output as a reception signal for Doppler measurement from the transmission and reception circuit 12 to the Doppler measurement portion 18.

The Doppler measurement portion 18 analyzes a Doppler shift frequency of a reception signal acquired for each ultrasound beam direction, and determines an ultrasound beam direction component of the bloodstream velocity at each position on each ultrasound beam (hereinafter, the component will also be referred to as a "Doppler measurement component"). The Doppler measurement portion 18 executes, for example, a correlation calculation between a signal section, of the reception signal, in a time range corresponding to a beam direction depth of the measurement position and the transmission signal, to determine the Doppler shift frequency at each position on the ultrasound beam, and determines the Doppler measurement component based on the Doppler shift frequency at each position. The Doppler measurement portion 18 outputs the Doppler measurement component to the signal processor 20.

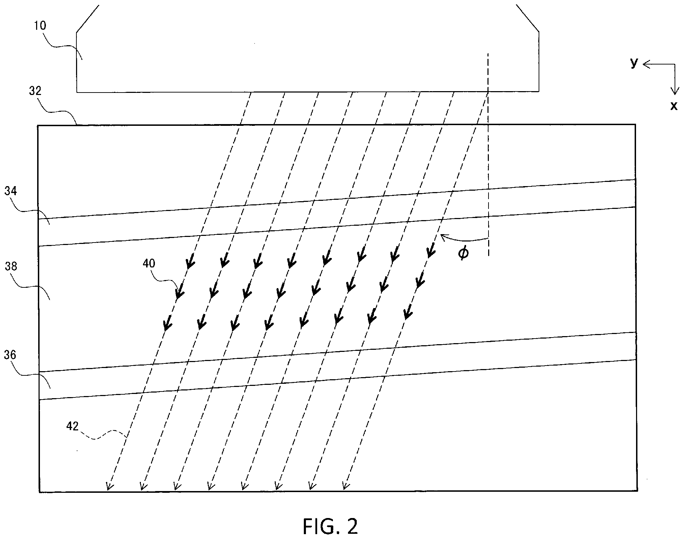

FIG. 2 conceptually shows a relationship between a tomographic image 32 and a Doppler measurement component 40. In the example configuration, a Doppler measurement ultrasound beam 42 formed by the probe 10 is tilted by an angle φ with respect to a positive x-axis direction, and the ultrasound beam 42 is linearly scanned in the y-axis direction. On the tomographic image 32, images of a near wall 34 and a far wall 36 of a blood vessel appear. A region sandwiched between the near wall 34 and the far wall 36 is a vascular lumen 38. The Doppler measurement ultrasound beam 42 is set at a direction not perpendicular to a longitudinal direction of the blood vessel, and is linearly scanned along the longitudinal direction of the blood vessel. FIG. 2 conceptually shows with an arrow each Doppler measurement component 40 determined by the Doppler measurement.

Although the Doppler measurement portion 18 of FIG. 1 determines the Doppler measurement component, the Doppler measurement portion 18 cannot determine a component in a direction orthogonal to the Doppler measurement ultrasound beam. Thus, the signal processor 20 determines the component of the bloodstream velocity in the direction orthogonal to the Doppler measurement ultrasound beam by integration based on the law of conservation of mass to be described below, based on a plurality of tomographic image data which are sequentially output with elapse of time from the tomographic image data producer 16, and the Doppler measurement component at each position.

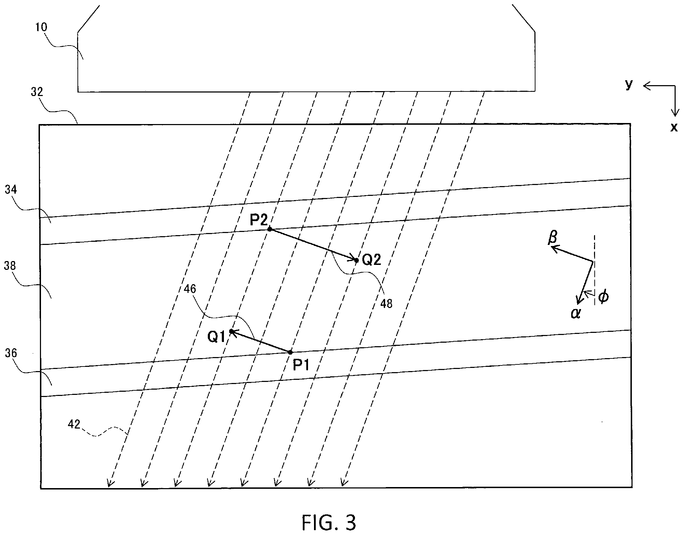

FIG. 3 conceptually shows a process in the integration based on the law of conservation of mass. In the integration based on the law of conservation of mass, an α-axis is determined in the Doppler measurement ultrasound beam direction and a β-axis is determines in a direction orthogonal to the Doppler measurement ultrasound beam. In the example configuration of FIG. 3, the Doppler measurement ultrasound beam direction is tilted by an angle φ with respect to the x-axis direction (vertical direction).

A component $V_\beta$ of the bloodstream velocity in a direction orthogonal to the Doppler measurement ultrasound beam (hereinafter referred to as "β-axis direction component") is determined by integrating, in the β-axis direction, an amount of change of the Doppler measurement component $V_\alpha$ in the α-axis direction ($\partial V_\alpha / \partial \alpha$). Thus, the β-axis direction component $V_\beta(Q)$ at a point Q is represented by the following Equation 1.

[Equation 1]

$$V_\beta(Q) = -\int_P^Q \frac{\partial V_\alpha}{\partial \alpha} d\beta + V_\beta(P)$$

Equation 1 is derived based on the law of conservation of mass that a flow amount of the blood flowing into an infinitesimal region having vertical and horizontal lengths of dα and dβ, respectively, and a flow amount of the blood flowing out of the infinitesimal region are equal to each other. In other words, Equation 1 is obtained by integrating with respect to β an equation setting the divergence of the bloodstream velocity (div$\vec{V}$) equal to 0, and is called an equation of continuity. An integration start position P is a position on the near wall surface or on the far wall surface. When it is possible to set a position on the near wall surface as the integration start position, a point on the near wall surface may be set as the integration start position P, and when it is possible to set a position on the far wall surface as the integration start position, a point on the far wall surface may be set as the integration start position P. The right side of Equation 1 is represented with partial differentiation and integration, but in the actual calculation, the calculation is done by adding and summing a difference of $V_\alpha$ at each position on the integration path.

For example, a β-axis direction component $V_\beta(Q1)$ at a point Q1 of FIG. 3 is determined by integrating the amount of change of the Doppler measurement component $V_\alpha$ in the α-axis direction along an integration path 46 in the positive β-axis direction to the point Q1, with a position P1 on the far wall surface being set as the integration start position. Similarly, the β-axis direction component $V_\beta(Q2)$ at a point Q2 is determined by integrating the amount of change of the Doppler measurement component $V_\alpha$ in the α-axis direction along an integration path 48 in the negative β-axis direction to the point Q2, with a point P2 on the near wall surface being set as the integration start position.

When the signal processor 20 of FIG. 1 determines these integration values, a β-axis direction component $V_\beta(P)$ of the motion velocity at the integration start position P is necessary as an initial value of the integration. Thus, the signal processor 20 determines, as the initial value of the integration, a β-axis direction component of the motion velocity of the integration start position P based on a plurality of tomographic image data which are sequentially output with the elapse of time from the tomographic image data producer 16. Then, integration based on the law of conservation of mass is executed using the determined initial value of integration, to determine the $\beta$-axis direction component $V_\beta(Q)$ at each position Q in the vascular lumen.

A specific process will now be described in which the signal processor 20 determines the bloodstream velocity based on the integration based on the law of conservation of mass, and displays, with figures, the bloodstream velocity on the display 30 along with the tomographic image. As a presumption of the process, the controller 14, the transmission and reception circuit 12, the probe 10, and the tomographic image data generator 16 repeatedly execute the process of producing the tomographic image data by the B-mode measurement. With this process, the tomographic image data producer 16 sequentially outputs a plurality of tomographic image data with elapse of time to the signal processor 20.

The signal processor 20 displays the tomographic image on the display 30 as a video image based on the tomographic image data which are sequentially output from the tomographic image data producer 16. In addition, the signal processor 20 may display the tomographic image on the display 30 as a still image based on data for one tomographic image.

A motion detector 22 extracts a pattern of a blood vessel wall surface from each tomographic image indicated by the plurality of tomographic image data, and determines the motion velocity of the blood vessel wall surface based on the pattern of the blood vessel wall surface extracted from each of the plurality of tomographic images. In other words, the motion detector 22 executes a pattern recognition process on the plurality of tomographic image data for a plurality of images in the past, and extracts the pattern of the blood vessel wall surface in each tomographic image. The motion detector 22 sets an integration start position on the pattern of the blood vessel wall surface, and determines the $\beta$-axis direction component of the motion velocity at the integration start position as the initial value of the integration based on the integration start position which is set for each tomographic image. This process is executed, for example, by tracking the pattern of the blood vessel wall surface based on a pattern matching of two tomographic image data produced at earlier and later times, and determining the motion velocity of the integration start position acquired for each of the plurality of tomographic image data. A velocity calculator 24 executes integration based on the law of conservation of mass using the determined initial value of integration, and determines the $\beta$-axis direction component at each position on the integration path.

The motion detector 22 sets a plurality of integration start positions along each of the near wall surface and the far wall surface, and the velocity calculator 24 executes the integration based on the law of conservation of mass from each integration start position. With this process, the $\beta$-axis direction component at each position of the vascular lumen is determined.

In the vascular lumen, there exist positions in which two $\beta$-axis direction components are determined; that is, a $\beta$-axis direction component $V_F$ determined based on the integration with a point on the near wall surface being set as the integration start position, and a $\beta$-axis direction component $V_B$ determined based on the integration with a point on the far wall surface being set as the integration start position. In this case, similar to the process disclosed in Patent Document 1, the $\beta$-axis direction component $V_\beta$ may be determined based on a weighted sum (weighted combination) according to the following Equation 2.

[Equation 2]

$$V_\beta = \omega(\beta)V_F + [1 - \omega(\beta)]V_B$$

Here, $\omega(\beta)$ is a weighting function. $\omega(\beta)$ is, for example, an increasing function related to $\beta$, and has a value of 0 at the integration start position on the far wall surface and a value of 1 at the integration start position on the near wall surface.

With such a process, a bloodstream velocity represented by the Doppler measurement component and the $\beta$-axis direction component is determined for each position of the vascular lumen.

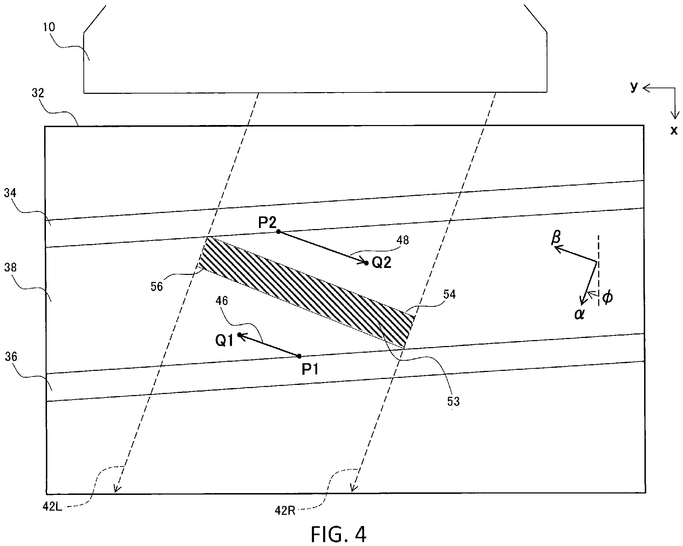

For a region in which the Doppler measurement ultrasound beam does not pass, the bloodstream velocity is not determined. In addition, even for a region where the Doppler measurement ultrasound beam passes, if the integration start position of the integration based on the law of conservation of mass is not determined and the integration path does not extend through, the $\beta$-axis direction component is not determined, and, consequently, the bloodstream velocity is not determined. Specifically, as shown in FIG. 4, in a region of an approximate triangle at the upper side, surrounded by the near wall surface, an integration path 54, and a Doppler measurement ultrasound beam 42R at the right end, the bloodstream velocity is determined. Similarly, in a region of an approximate triangle at the lower side, surrounded by the far wall surface, an integration path 56, and a Doppler measurement ultrasound beam 42L at the left end, the bloodstream velocity is determined. However, for an incomplete region 53 surrounded by the integration path 54, the integration path 56, the left-end Doppler measurement ultrasound beam 42L, and the right-end Doppler measurement ultrasound beam 42R, although the Doppler measurement ultrasound beam passes this region, the path of the integration based on the law of conservation of mass does not extend through this region, and thus, the bloodstream velocity is not determined.

In consideration of this, the ultrasound diagnostic apparatus determines the bloodstream velocity in the incomplete region 53 by a sub-beam method to be described below. In the sub-beam method, ultrasound which forms a sub-beam is transmitted and received separately from the Doppler measurement ultrasound beam serving as the main beam, to acquire the initial condition of the integration based on the law of conservation of mass, and to determine the bloodstream velocity in the incomplete region.

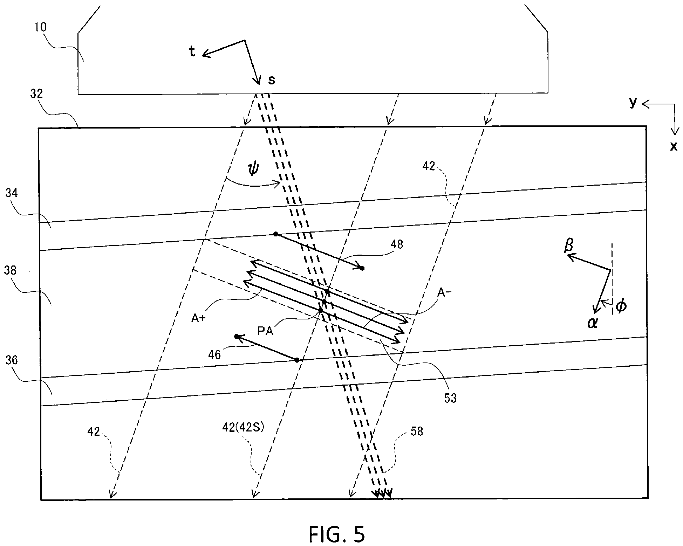

FIG. 5 is a diagram explaining the principle of the sub-beam method. In the probe 10, ultrasound is transmitted and received form a plurality of sub-beams 58 arranged in a matched direction, separately from the Doppler measurement ultrasound beam 42. Each sub-beam 58 has a direction different from the direction of the Doppler measurement ultrasound beam 42, and passes through the incomplete region 53. Here, it is assumed that an angle formed by the Doppler measurement ultrasound beam 42 and the sub-beam is $\psi$. In addition, a coordinate axis in a same direction as the direction of the sub-beams 58 is set as an s-axis, and a coordinate axis in a direction orthogonal to the s-axis is set as a t-axis.

In the sub-beam method, an integration start position PA is set at each of intersections between the plurality of sub-beams 58 and an integration start position beam 42S which is one of the plurality of Doppler measurement ultrasound beams 42. In addition, based on the transmission and reception of the ultrasound forming the sub-beams 58, the sub-beam direction component at each integration start position PA is Doppler-measured. Further, an amount of change of the Doppler measurement component $V_\alpha$ in the $\alpha$-axis direction is integrated along an integration path A+ from each integration start position PA toward the positive $\beta$-axis direction. With the integration calculation in the positive direction based on the law of conservation of mass (first integration calculation), the $\beta$-axis direction component at each point on the integration path A+ is determined. In addition, an amount of change of the Doppler measurement component $V_\alpha$ in the $\alpha$-axis direction is integrated along an integration path A- from the integration start position PA toward the negative $\beta$-axis direction. With the integration calculation in the negative direction based on the law of conservation of mass (second integration calculation), the $\beta$-axis direction component at each point on the integration path A- is determined.

The initial value of each of the positive direction integration calculation and the negative direction integration calculation is the $\beta$-axis direction component at the integration start position PA. The $\beta$-axis direction component is determined as follows based on the Doppler measurement component and the sub-beam direction component at the integration start position PA.

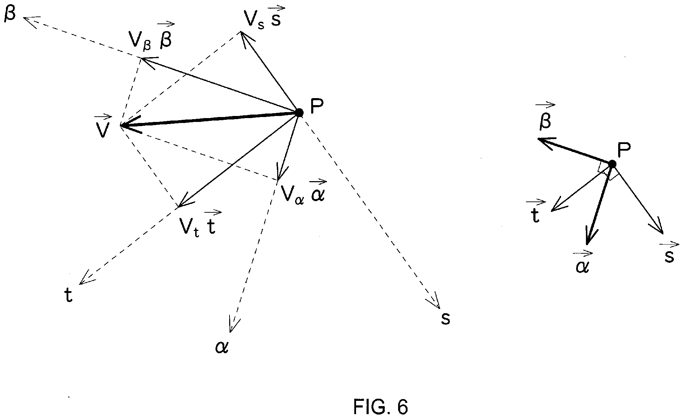

On a right side of FIG. 6, unit vectors $\vec{\alpha}$ and $\vec{\beta}$ corresponding to the $\alpha$-axis and the $\beta$-axis, and unit vectors $\vec{s}$ and $\vec{t}$ corresponding to the s-axis and the t-axis are shown. On a left side of FIG. 6, a Doppler measurement component $V_\alpha\vec{\alpha}$ measured based on the Doppler measurement ultrasound beam, and the sub-beam direction component $V_s\vec{s}$ measured based on the sub-beam are shown. In addition, a $\beta$-axis direction component $V_\beta\vec{\beta}$ and a sub-beam orthogonal direction component $V_t\vec{t}$ are shown as unknown vector components. The components $V_\alpha$, $V_\beta$, $V_s$, and $V_t$ are in the relationship shown in the following Equation 3.

[Equation 3]

$$\begin{pmatrix} V_\alpha \\ V_\beta \end{pmatrix} = [T] \begin{pmatrix} V_s \\ V_t \end{pmatrix}$$

Here, the matrix [T] is a coordinate conversion matrix (rotation matrix of a rotational angle $\psi$) which converts the value in the st coordinate system into a value in the $\alpha\beta$ coordinate system. The origins of the st coordinate system and the $\alpha\beta$ coordinate system are common at the integration start position. The coordinate conversion matrix [T] is known, and the Doppler measurement component $V_\alpha$ and the sub-beam direction component $V_s$ are determined by the Doppler measurement. Therefore, by solving Equation 3 for the $\beta$-axis direction component $V_\beta$, the $\beta$-axis direction component $V_\beta$ is determined as an initial value of each of the positive direction integration calculation and the negative direction integration calculation.

This calculation will now be described according to the drawing on the left side of FIG. 6. The Doppler measurement component $V_\alpha\vec{\alpha}$ and the sub-beam direction component $V_s\vec{s}$ are determined by the Doppler measurement in advance. Using the Doppler measurement component $V_\alpha\vec{\alpha}$ and the sub-beam direction component $V_s\vec{s}$, $V_\beta\vec{\beta}$ and $V_t\vec{t}$ are determined such that $\vec{V} = V_\alpha\vec{\alpha} + V_\beta\vec{\beta}$ and $\vec{V} = V_s\vec{s}$ and $V_t\vec{t}$ are equal to each other, to determine the $\beta$-axis direction component $V_\beta$ as the initial value.

With such a process, the $\beta$-axis direction component is determined for each point in the incomplete region. A vector in which the Doppler measurement component and the $\beta$-axis direction component determined for each point in the incomplete region are combined is the bloodstream velocity at each point.

The process for the ultrasound diagnostic apparatus to determine the bloodstream velocity of the incomplete region based on the sub-beam method will now be described with reference back to FIG. 1. First, the ultrasound diagnostic apparatus determines the bloodstream velocity by the above-described process for each position of regions other than the incomplete region, based on transmission and reception of ultrasound forming the B-mode measurement ultrasound beam and the Doppler measurement ultrasound beam.

The controller 14 controls the transmission and reception circuit 12 to form a plurality of sub-beams by an ultrasound transmitted from the probe 10, by a process similar to the process of forming the Doppler measurement ultrasound beam. Because the plurality of sub-beams are arranged with a matching direction, the plurality of sub-beams may alternatively be formed by linearly scanning one sub-beam. In addition, the transmission and reception circuit 12 produces a reception signal for each sub-beam direction according to the control by the controller 14, and outputs the signal to the Doppler measurement portion 18. The Doppler measurement portion 18 analyzes the Doppler shift frequency of each reception signal acquired for each sub-beam direction, and determines the sub-beam direction component at the integration start position which is an intersection between each sub-beam and the integration start position beam. The Doppler measurement portion 18 outputs the sub-beam direction component at each integration start position to the signal processor 20.

A sub-beam method calculator 26 determines the bloodstream velocity for each point in the incomplete region based on the sub-beam method. That is, the sub-beam method calculator 26 determines, as the initial value, the $\beta$-axis direction component for each integration start position based on the Doppler measurement component and the sub-beam direction component at each integration start position. The positive direction integration calculation and the negative direction integration calculation are executed for each integration start position, and the $\beta$-axis direction component is determined for each point in the incomplete region. The sub-beam method calculator 26 sets, as the bloodstream velocity at each point, a vector in which the Doppler measurement component determined in advance for each point in the incomplete region and the $\beta$-axis direction component determined for each point by the sub-beam method are combined.

A display image former 28 produces bloodstream velocity data for displaying the bloodstream velocity at each position in the vascular lumen with figures such as an arrow, and displays on the display 30 an image in which the figure showing the bloodstream velocity is overlapped on the tomographic image.

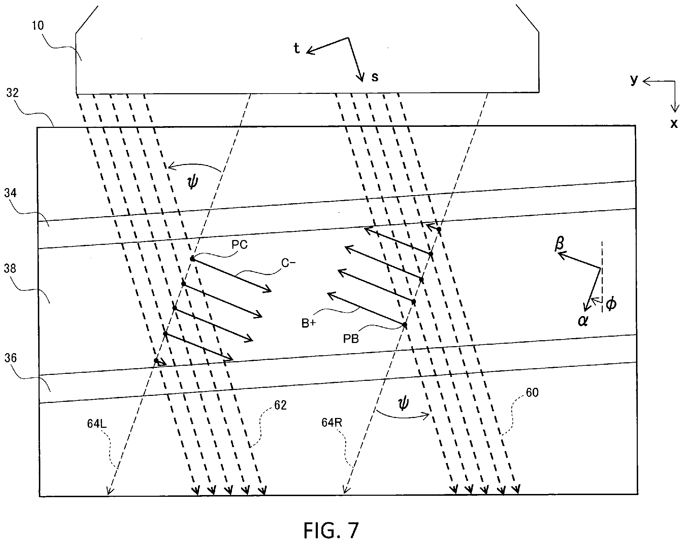

Next, a two-end beam method which is an advanced application of the sub-beam method will be described. In this method, two Doppler measurement ultrasound beams at the right end and the left end are set as integration start position beams. As shown in FIG. 7, in the two-end beam method, a plurality of first sub-beams 60 intersecting a right-end integration start position beam 64R are formed. The plurality of first sub-beams 60 are arranged with a matching direction. At each of the intersections between the plurality of first sub-beams 60 and the right-end integration start position beam 64R, an integration start position PB is set. In addition, based on the transmission and reception of the ultrasound forming each first sub-beam 60, the first sub-beam direction component at each integration start position PB is Doppler-measured. Further, an amount of change of the Doppler measurement component $V_\alpha$ in the $\alpha$-axis direction is integrated along an integration path B+ from each integration start position PB toward the positive $\beta$-axis direction. With the positive direction integration calculation based on the law of conservation of mass, the $\beta$-axis direction component at each point on the integration path B+ is determined.

The initial value of the positive direction integration calculation is the $\beta$-axis direction component at the integration start position PB. The $\beta$-axis direction component is determined by the vector calculation explained with reference to FIG. 6, based on the Doppler measurement component and the first sub-beam direction component at the integration start position PB.

Further, in the two-end beam method, a plurality of second sub-beams 62 which intersect a left-end integration start beam 64L are formed. The plurality of second sub-beams 62 are arranged with a matching direction. At each of the intersections of the plurality of second sub-beams 62 and the left-end integration start position beam 64L, an integration start position PC is set. In addition, a second sub-beam direction component at each integration start position PC is Doppler-measured based on transmission and reception of ultrasound for forming each second sub-beam 62. Moreover, an amount of change of the Doppler measurement component $V_\alpha$ in the $\alpha$-axis direction is integrated along an integration path C- from each integration start position PC toward a negative $\beta$-axis direction. With the negative direction integration calculation based on the law of conservation of mass, the $\beta$-axis direction component at each point on the integration path C- is determined.

The initial value of the negative direction integration calculation is the $\beta$-axis direction component at the integration start position PC. The $\beta$-axis direction component is calculated by the vector calculation explained above with reference to FIG. 6, based on the Doppler measurement component and the second sub-beam direction component at the integration start position PC.

In a region sandwiched between the left and right integration start position beams, there exist positions where two $\beta$-axis direction components are determined; that is, a $\beta$-axis direction component $V_{\beta+}$ determined based on the positive direction integration calculation and a β-axis direction component $V_{\beta-}$ determined based on the negative direction integration calculation. In this case, similar to the process disclosed in Patent Document 1, the β-axis direction component $V_\beta$ may be determined based on a weighted addition according to the following Equation 4.

[Equation 4]

$$V_\beta = \omega(\beta)V_{\beta-} + [1 - \omega(\beta)]V_{\beta+}$$

Here, $\omega(\beta)$ is a weighting function. $\omega(\beta)$ is, for example, an increasing function related to β, and has a value of 0 at the integration start position PB on the right-end integration start position beam 64R and a value of 1 at the integration start position PC on the left-end integration start position beam 64L.

With such a process, the β-axis direction component is determined for each point in the region sandwiched between the left and right integration start position beams. A vector in which the Doppler measurement component and the β-axis direction component determined for each point in the region are combined is set as the bloodstream velocity at each point.

According to the two-end beam method, it is not necessary to set the blood vessel wall surface as the integration start position for the integration based on the law of conservation of mass. Therefore, the initial condition of the integration based on the law of conservation of mass can be determined without executing tracking of the pattern of the blood vessel wall surface based on the tomographic image data or the like.

The process executed by the ultrasound diagnostic apparatus based on the two-end beam method is similar to the sub-beam method explained above with reference to FIG. 5. That is, the probe 10, the transmission and reception circuit 12, the controller 14, and the Doppler measurement portion 18 execute the Doppler measurement based on each first sub-beam and each second sub-beam, and the sub-beam method calculator 26 executes the integration based on the law of conservation of mass for each integration start position which is set on each integration start position beam.

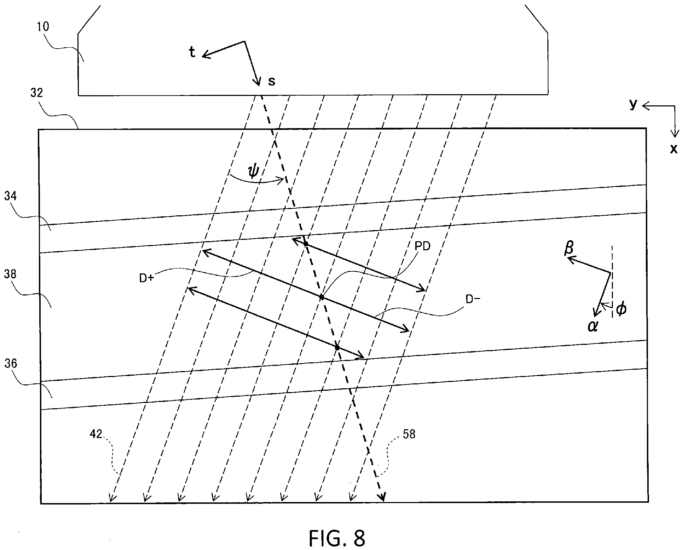

In the above, an embodiment is explained which uses a plurality of sub-beams, but alternatively, the number of sub-beams may be 1. FIG. 8 is a diagram for explaining the principle of a single sub-beam method which uses one sub-beam 58.

In the single sub-beam method, in a region in which the Doppler measurement ultrasound beam 42 is scanned, an integration start position PD is set at each of intersections between the sub-beam 58 and the plurality of Doppler measurement ultrasound beams 42. In addition, based on transmission and reception of the ultrasound for forming the sub-beam 58, the sub-beam direction component is measured for each integration start position PD on the sub-beam 58.

An amount of change of the Doppler measurement component $V_\alpha$ in the α-axis direction is integrated from each integration start position PD along an integration path D+ toward the positive β-axis direction. With the positive direction integration calculation based on the law of conservation of mass, the β-axis direction component at each point on the integration path D+ is determined. In addition, an amount of change of the Doppler measurement component $V_\alpha$ in the α-axis direction is integrated from the integration start position PD along an integration path D- toward the negative β-axis direction. With the negative direction integration calculation based on the law of conservation of mass, the β-axis direction component at each point on the integration path D- is determined.

The initial value of each of the positive direction integration calculation and the negative direction integration calculation is the β-axis direction component at the integration start position PD. The β-axis direction component is determined by executing a vector calculation for the Doppler measurement component and the sub-beam direction component at the integration start position PD.

With such a process, the β-axis direction component of the bloodstream velocity is determined for each point in the vascular lumen. A vector in which the Doppler measurement component and the β-axis direction component determined for each point in the vascular lumen are combined is set as the bloodstream velocity at each point.

In this manner, in the sub-beam method, the Doppler measurement ultrasound beam serving as the main beam is scanned, and each Doppler measurement component (main beam direction component) is Doppler-measured based on the ultrasound received from each Doppler measurement ultrasound beam direction. In addition, a path of integration based on the law of conservation of mass is set in a direction intersecting the Doppler measurement ultrasound beam direction. Ultrasound forming the sub-beam passing through the intersecting integration path is transmitted and received, and, based on the ultrasound received from the sub-beam direction, the sub-beam direction component is Doppler-measured for a passing point of the sub-beam in the intersecting integration path. The sub-beam has a direction different from the direction of the main beam passing through the passing point, and the passing point is set as the integration start position. The initial value of the integration based on the law of conservation of mass is the component of the bloodstream velocity in the intersecting path direction, and is determined based on the Doppler measurement component and the sub-beam direction component at the passing point.

The number of sub-beams; that is, the number of integration paths, may be determined according to a necessary processing speed. For example, when a bloodstream velocity is to be determined at a larger number of points, a large number of sub-beams may be used, and, when a higher speed process is necessary, the number of sub-beams may be reduced.

The passing point of the sub-beam on the intersecting integration path is one end, the other end, or a partway point of the intersecting integration path. The integration based on the law of conservation of mass is executed from the passing point serving as the integration start position and along the intersecting integration path. When the passing point is a partway point of the intersecting integration path, integration is executed in one direction away from the partway point along the intersecting integration path, and integration is executed in the other direction away from the partway point along the intersecting integration path.

As described above, the ultrasound diagnostic apparatus according to the present disclosure executes the wall-surface method having the wall surface of the circulatory organ as the integration start position and the sub-beam method having the point on the integration start position beam as the integration start position.

As exemplified in FIGs. 5, 7, and 8, the ultrasound diagnostic apparatus of the present disclosure uses one of the wall-surface method and the sub-beam method or combines these methods, so that the apparatus can determine the bloodstream velocity in the circulatory organ for various shapes of the circulatory organs. Here, other example configurations which are not described above will be described.

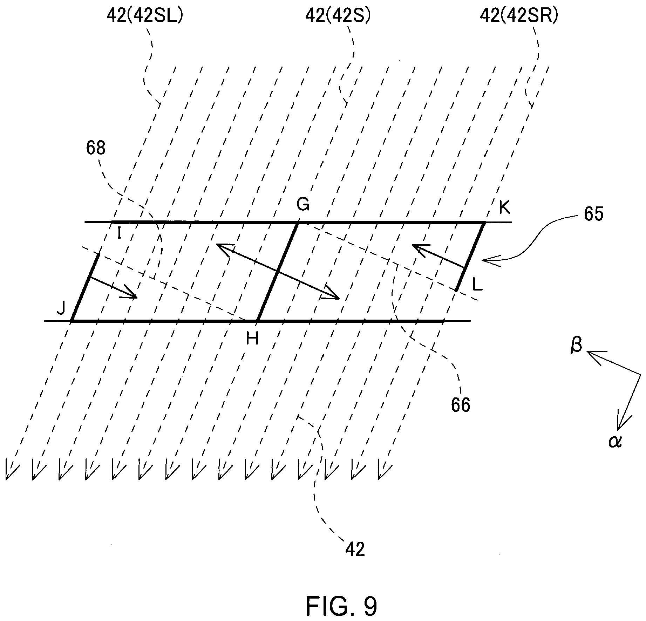

FIG. 9 shows an example configuration in which the bloodstream velocity in the vascular lumen is determined by only the sub-beam method. The Doppler measurement ultrasound beam 42 is set at a direction not perpendicular to the longitudinal direction of a blood vessel 65, and is linearly scanned along the longitudinal direction of the blood vessel 65. Of the straight lines showing the integration start position beams 42S, a plurality of sub-beams (not shown) arranged with a matching direction intersect a line segment GH which is a portion of the vascular lumen, and each of intersections between the line segment GH and the plurality of sub-beams is set as the integration start position. From each integration start position, a positive direction integration calculation is executed in the positive β-axis direction, and a negative direction integration calculation is executed in the negative β-axis direction. With this process, the β-axis direction component is determined for a region sandwiched between a virtual straight line 66 extending from a point G toward the negative β-axis direction and a virtual straight line 68 extending from a point H toward the positive β-axis direction.

Of the straight lines showing the left-end integration start position beams 42SL, in a line segment IJ which is a left side portion of the virtual straight line 68 and passing through the vascular lumen, a plurality of integration start positions in the sub-beam method are set. From each integration start position, the negative direction integration calculation is executed in the negative β-axis direction. With this process, the β-axis direction component is determined for a region surrounded by the line segment IJ, the virtual straight line 68, and the far wall surface.

Of the straight lines showing the right-end integration start position beams 42SR, in a line segment KL which is a right side portion of a virtual straight line 66 and passing through the vascular lumen, a plurality of integration start positions in the sub-beam method are set. From each integration start position, the positive direction integration calculation is executed in the positive β-axis direction. With this process, the β-axis direction component is determined for a region surrounded by the line segment KL, the virtual straight line 66, and the near wall surface.

The β-axis direction component in the vascular lumen determined in this manner and the Doppler measurement component determined by the Doppler measurement ultrasound beam 42 are combined, to determine the bloodstream velocity in the vascular lumen.

Depending on an angle of intersection of the Doppler measurement ultrasound beam and the blood vessel, the size of the vascular lumen, or the like, an incomplete region may be formed in which the bloodstream velocity cannot be determined with the sub-beam method alone. In this case, there may be cases where the bloodstream velocity in the incomplete region can be determined by combination with the wall-surface method.

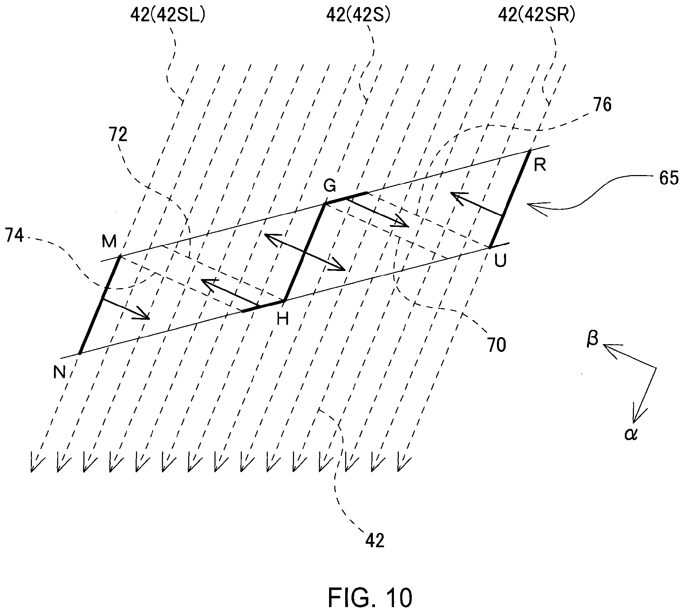

FIG. 10 shows an example configuration in which the sub-beam method and the wall-surface method are combined to determine the bloodstream velocity of the vascular lumen. Of the straight lines showing the integration start position beams 42S, on a line segment GH which is a portion of the vascular lumen, a plurality of integration start positions in the sub-beam method are set. From each integration start position, a positive direction integration calculation is executed in the positive β-axis direction, and a negative direction integration calculation is executed in the negative β-taxis direction. With this process, the β-axis direction component is determined in a region sandwiched between a virtual straight line 70 extending from a point G toward the negative β-axis direction and a virtual straight line 72 extending from a point H toward the positive β-axis direction.

Of the straight lines showing the left-end integration start position beams 42SL, on a line segment MN which is a portion of the vascular lumen, a plurality of integration start positions in the sub-beam method are set. From each integration start position, a negative direction integration calculation is executed in the negative β-axis direction. With this process, a β-axis direction component is determined for a region surrounded by a virtual straight line 74 extending from a point M toward the negative β-axis direction, the line segment MN, and the far wall surface.

Of the straight lines showing the right-end integration start position beams 42SR, on a line segment RU which is a portion of the vascular lumen, a plurality of integration start positions for the sub-beam method are set. From each integration start position, a positive direction integration calculation is executed in the positive β-axis direction. With this process, a β-axis direction component is determined for a region surrounded by a virtual straight line 76 extending from a point U toward the positive β-axis direction, the line segment RU, and the near wall surface.

For a region sandwiched between the virtual straight line 72 and the virtual straight line 74, a plurality of integration start positions for the wall-surface method are set on the far wall surface. From each integration start position, a positive direction integration calculation is executed in the positive β-axis direction. With this process, a β- axis direction component is determined for a region sandwiched between the virtual straight line 72 and the virtual straight line 74.

For a region sandwiched between the virtual straight line 70 and the virtual straight line 76, a plurality of integration start positions for the wall-surface method are set on the near wall surface. From each integration start position, a negative direction integration calculation is executed in the negative β-axis direction. With this process, a β-axis direction component is determined for the region sandwiched between the virtual straight line 70 and the virtual straight line 76.

The β-axis direction component in the vascular lumen determined in this manner and the Doppler measurement component determined with the Doppler measurement ultrasound beam 42 are combined, to determine the bloodstream velocity in the vascular lumen.

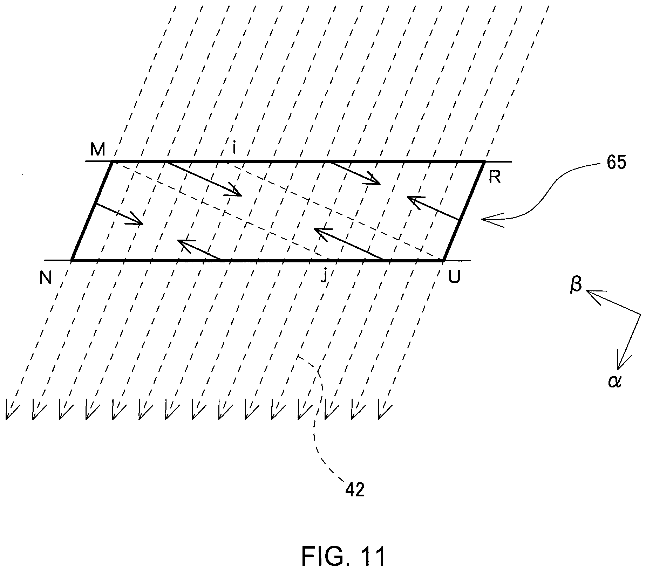

In an example configuration shown in FIG. 11, two β-axis direction components are determined for each region, and the weighted summing of the two β-axis direction components is executed based on Equation 2 or Equation 4, to determine the β-axis direction component.

In a region surrounded by a virtual straight line Mj extending from a point M in the negative β-axis direction to the far wall surface, the line segment MN, and the far wall surface, two β-axis direction components are determined based on the negative direction integration calculation for each of a plurality of integration start positions on the line segment MN and the positive direction integration calculation for each of the plurality of integration start positions on the far wall surface. Each integration start position on the line segment MN is an integration start position based on the sub-beam method, and each integration start position on the far wall surface is an integration start position based on the wall-surface method.

Further, in a region surrounded by a virtual straight line Ui extending from a point U in the positive β-axis direction to the near wall surface, the line segment RU, and the near wall surface, two β-axis direction components are determined based on the positive direction integration calculation for each of a plurality of integration start positions on the line segment RU and the negative direction integration calculation for each of the plurality of integration start positions on the near wall surface. Each integration start position on the line segment RU is an integration start position based on the sub-beam method, and each integration start position on the near wall surface is an integration start position based on the wall-surface method.

Moreover, in a region sandwiched between the virtual straight line Mj and the virtual straight line Ui, two β-axis direction components are determined based on the negative direction integration calculation for each of a plurality of integration start positions on the near wall surface and the positive direction integration calculation for each of a plurality of integration start positions on the far wall surface. Each integration start position is an integration start position based on the wall-surface method.

The β-axis direction component in the vascular lumen determined in this manner and the Doppler measurement component determined with the Doppler measurement ultrasound beam 42 are combined, to determine the bloodstream velocity in the vascular lumen.

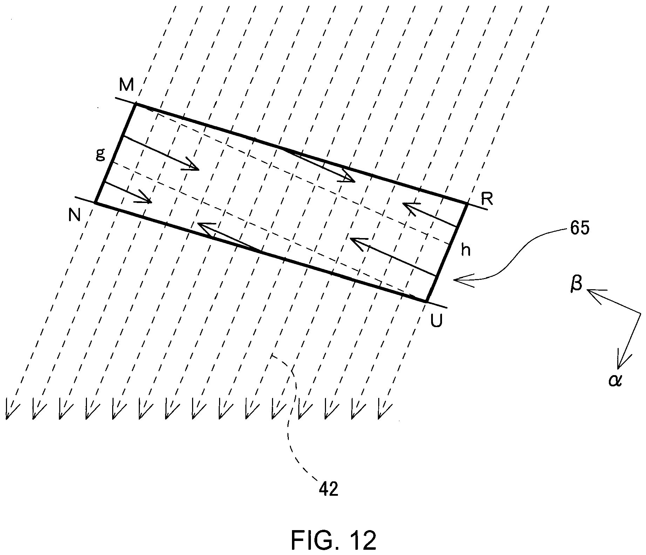

In an example configuration shown in FIG. 12 also, two β-axis direction components are determined in each region, and the weighted summing is executed on the two β-axis direction components based on Equation 2 or Equation 4, to determine the β-axis direction component.

In a region surrounded by a virtual straight line Mh extending from a point M in the negative β-axis direction to the line segment RU, a line segment Rh, and the near wall surface, two β-axis direction components are determined based on the positive direction integration calculation for each of a plurality of integration start positions on the line segment Rh and the negative direction integration calculation for each of a plurality of integration start positions on the near wall surface. Each integration start position on the line segment Rh is an integration start position based on the sub-beam method, and each integration start position on the near wall surface is an integration start position based on the wall-surface method.

In addition, in a region surrounded by a virtual straight line Ug extending from a point U in the positive β-axis direction to the line segment MN, a line segment gN, and the far wall surface, two β-axis direction components are determined based on the negative direction integration calculation for each of a plurality of integration start positions on the line segment gN and the positive direction integration calculation for each of a plurality of integration start positions on the far wall surface. The plurality of integration start positions on the line segment gN are integration start positions based on the sub-beam method, and the plurality of integration start positions on the far wall surface are integration start positions based on the wall-surface method.

Further, in a region sandwiched between the virtual straight line Mh and the virtual straight line Ug, two β-axis direction components are determined based on the negative direction integration calculation for each of a plurality of integration start positions on a line segment Mg and the positive direction integration calculation for each of a plurality of integration start positions on a line segment hU. Each integration start position is an integration start position based on the sub-beam method.

The β-axis direction component in the vascular lumen determined in this manner and the Doppler measurement component determined by the Doppler measurement ultrasound beam 42 are combined, to determine the bloodstream velocity in the vascular lumen.

In the above description, an embodiment is described in which one integration start position is set for an intersection between one integration start position beam and one sub-beam. As an alternative to such a setting of the integration start position, two or more sub-beams that intersect at a position to be set as the integration start position may be used. The directions of the two or more sub-beams differ from the direction of the Doppler measurement ultrasound beam (main beam) and from the direction perpendicular to the longitudinal direction of the blood vessel. For example, when two sub-beams are used, an integration start position is set at an intersection between a first sub-beam and the integration start position beam. Then, a first provisional initial value of integration based on the law of conservation of mass is determined based on the Doppler measurement component at the integration start position and the bloodstream velocity component in the direction of the first sub-beam. Further, a second provisional initial value of the integration based on the law of conservation of mass is determined based on the Doppler measurement component at the integration start position and a bloodstream velocity component in a direction of the additional, second sub-beam. The initial value of the integration based on the law of conservation of mass is determined by an average value of the first provisional initial value and the second provisional initial value, a weighted average of the provisional initial values taking into consideration the importance of each provisional initial value, or the like.

Depending on an angular relationship among the sub-beam, the integration start position beam, and the integration path, an error in the initial value at the integration start position may become significant. Using two or more sub-beams which intersect at the point of the integration start position, such an error may be reduced.

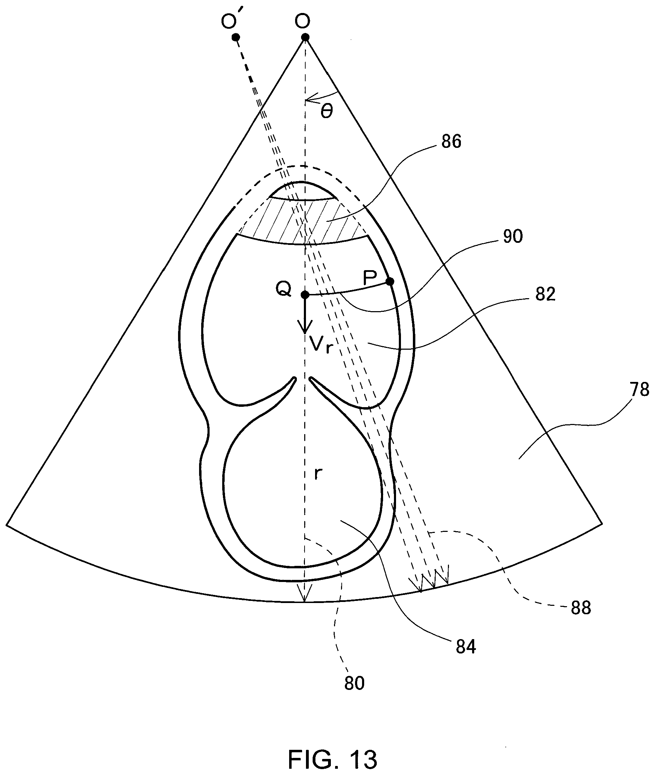

Next, an embodiment will be described in which the ultrasound beam is sector-scanned. The sector scanning is a scanning method in which the ultrasound beam is reciprocated to change the ultrasound beam direction. FIG. 13 conceptually shows a tomographic image 78 and a Doppler measurement ultrasound beam 80 in the sector scanning. The tomographic image 78 shows the left atrium 84 and a left ventricle 82. Of wall surfaces of the left ventricle 82, a portion shown with a broken line is a portion where an image is not acquired because the measurement condition is inferior. In FIG. 13, a direction of the Doppler measurement ultrasound beam 80 is set as an r-axis direction, and a direction orthogonal to the Doppler measurement ultrasound beam 80 is set as a θ-axis direction.

The B-mode measurement ultrasound beam (not shown) is reciprocated around a transmission and reception point O by the sector scanning. Based on the ultrasound received from each direction of the B-mode measurement ultrasound beam, tomographic image data are produced. The Doppler measurement ultrasound beam 80 is also reciprocated around the transmission and reception point O by the sector scanning, and, based on the ultrasound received from each direction of the Doppler measurement ultrasound beam 80, a Doppler measurement component $V_r$ (r-axis direction component) at each position on the Doppler measurement ultrasound beam is determined. A θ-axis direction component Ve at each position on the ultrasound beam 80 is determined by an integration based on the law of conservation of mass along the θ-axis direction. An integration start position of the integration is set at a plurality of positions on the heart wall surface. A point P in FIG. 13 shows one of the plurality of integration start positions. In addition, an initial value of the integration is acquired by determining a motion velocity of each integration start position based on the tomographic image data sequentially acquired with the elapse of time. The Doppler measurement component $V_r$ and the θ-axis direction component Ve determined in this manner are combined, to determine the bloodstream velocity.

Of the wall surface of the left ventricle 82, in an incomplete region 86 sandwiched by portions in which an image is not acquired because the measurement condition is inferior, the bloodstream velocity is determined based on the sub-beam method using a plurality of sub-beams 88 extending from a transmission and reception point O'.

A process for the ultrasound diagnostic apparatus to determine the bloodstream velocity by sector scanning of the ultrasound beam will now be described. The controller 14 shown in FIG. 1 controls the transmission and reception circuit 12 to form, in a time divisional manner, the B-mode measurement ultrasound beam and the Doppler measurement ultrasound beam at the probe 10, and to sector scan the ultrasound beams to the target.

The tomographic image data producer 16 produces tomographic image data based on the reception signal which is output from the transmission and reception circuit 12 in response to the sector scanning of the B-mode measurement ultrasound beam, and outputs the produced data to the signal processor 20. The Doppler measurement portion 18 determines the Doppler measurement component at each position on each Doppler measurement ultrasound beam based on the reception signal which is output from the transmission and reception circuit 12 in response to the sector scanning of the Doppler measurement ultrasound beam, and outputs the determined component to the signal processor 20.

The θ-axis direction component Ve at each position on the Doppler measurement ultrasound beam is determined by integration based on the law of conservation of mass. The motion detector 22 executes a pattern recognition process on the plurality of tomographic image data of a plurality of images in the past, to extract a pattern of the heart wall surface on each tomographic image, and sets a plurality of integration start positions on the pattern of the heart wall surface. The motion detector 22 determines, as an initial value of the integration, the θ-axis direction component of the motion velocity of the integration start position, based on the integration start position which is set for each tomographic image.

The velocity calculator 24 executes the integration based on the law of conservation of mass in the θ-axis direction for the integration start position, as shown in FIG. 13, and determines the θ-axis direction component $V_\theta(Q)$ at a point Q on an integration path 90. More specifically, the θ-axis direction component $V_\theta(Q)$ at the point Q is determined by the following Equation 5.

[Equation 5]

$$V_\theta(Q) = -\int_P^Q \frac{\partial(rV_r)}{\partial r} d\theta + V_\theta(P) = -\int_P^Q \left(V_r + r\frac{\partial V_r}{\partial r}\right) d\theta + V_\theta(P)$$

Equation 5 shows, in a polar coordinate system, Equation 1 which is represented in the orthogonal coordinate system. An initial value Ve(P) of integration based on the law of conservation of mass is a θ-axis direction component of a motion velocity of the integration start position P, and is determined by the motion detector 22 as described above. A formula at the center and the right side of Equation 5 are represented by partial differentiation and integration, but in the actual calculation, a difference of ($r \cdot V_r$) at each position on the integration path 90 is added and summed.

In the incomplete region 86, although the Doppler measurement ultrasound beam 80 passes through, because the path of the integration based on the law of conservation of mass does not extend through, the θ-axis direction component is not determined. Thus, the ultrasound diagnostic apparatus executes a process based on the sub-beam method as described below.

The controller 14 shown in FIG. 1 controls the transmission and reception circuit 12, to form a plurality of sub-beams by ultrasound transmitted from the probe 10. The transmission and reception circuit 12 produces a reception signal for each sub-beam direction according to the control by the controller 14, and outputs the produced signal to the Doppler measurement portion 18.

The Doppler measurement portion 18 analyzes the Doppler shift frequency of the reception signal acquired for each sub-beam direction, and determines the sub-beam direction component at a predetermined position on each sub-beam in the incomplete region. The Doppler measurement portion 18 outputs the sub-beam direction component at each position to the signal processor 20.

Each sub-beam 88 shown in FIG. 13 has a direction different from the direction of the Doppler measurement ultrasound beam 80, extends from a transmission and reception point O' different from the transmission and reception point O, and passes through the incomplete region 86. Because each sub-beam 88 extends from a same transmission and reception point O', each sub-beam may be formed by sector scanning around the transmission and reception point O'. In the sub-beam method calculator, a process based on the sub-beam method as described below is executed.

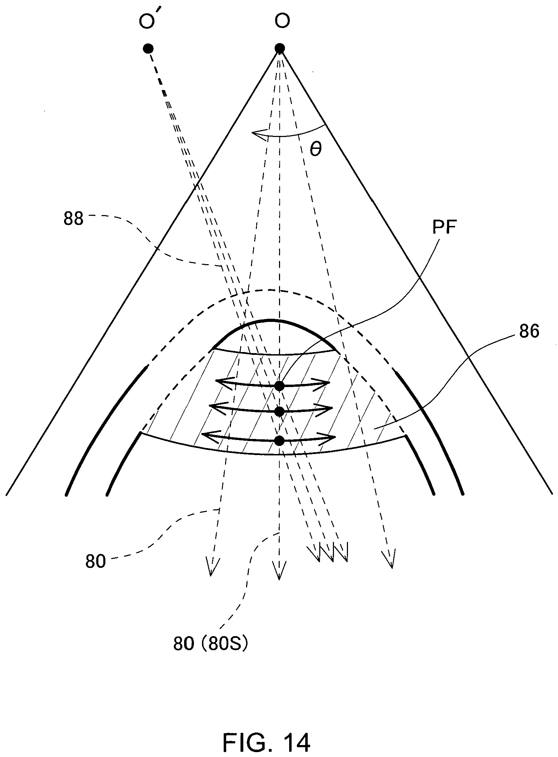

FIG. 14 shows an enlarged view of a region near the incomplete region 86. In the sub-beam method, an integration start position PF is set at each of intersections between the plurality of sub-beams 88 and an integration start position beam 80S which is one of the plurality of Doppler measurement ultrasound beams 80. For each integration start position PF, a θ-axis direction component of the bloodstream velocity is determined as the initial value, based on the Doppler measurement component and the sub-beam direction component. In addition, a positive direction integration calculation along the positive θ-axis direction and the negative direction integration calculation along the negative θ-axis direction are executed for each integration start position PF, and a θ-axis direction component of the bloodstream velocity is determined for each point in the incomplete region 86. A vector in which the Doppler measurement component and the θ-axis direction component determined for each point in the incomplete region 86 are combined is set as the bloodstream velocity at each point.

With such a process, the bloodstream velocity is determined for each position of the left atrium and the left ventricle. The display image former 28 of FIG. 1 produces bloodstream velocity data for displaying the bloodstream velocity at each position of the left atrium and the left ventricle with figures such as an arrow or the like, and displays, on the display 30, an image in which the figure showing the bloodstream velocity is overlapped with the tomographic image.

In the above description, the orthogonal coordinate system and the polar coordinate system are described. By coordinate-converting Equation 1, an integration based on the law of conservation of mass can be executed in any arbitrary coordinate system suitable for the shape of the circulatory organ. In this case, an initial value of the integration in the arbitrary coordinate system is determined using the Doppler measurement component and the sub-beam direction component at the integration start position. With this configuration, the integration based on the law of conservation of mass can be executed according to the integration path having a shape suitable for the shape of the circulatory organ.

REFERENCE SIGNS LIST

10 PROBE; 12 TRANSMISSION AND RECEPTION CIRCUIT; 14 CONTROLLER; 16 TOMOGRAPHIC IMAGE DATA PRODUCER; 18 DOPPLER MEASUREMENT PORTION; 20 SIGNAL PROCESSOR; 22 MOTION DETECTOR; 24 VELOCITY CALCULATOR; 26 SUB-BEAM METHOD CALCULATOR; 28 DISPLAY IMAGE FORMER; 30 DISPLAY; 32, 78 TOMOGRAPHIC IMAGE; 34 NEAR WALL; 36 FAR WALL; 38 VASCULAR LUMEN; 40 DOPPLER MEASUREMENT COMPONENT; 42, 80 DOPPLER MEASUREMENT ULTRASOUND BEAM; 42S, 42SR, 42SL, 64R, 64L INTEGRATION START POSITION BEAM; 46, 48, 54, 56, 90 A+, A-, B+, C-, D+, D-INTEGRATION PATH; 53, 86 INCOMPLETE REGION; 58, 88 SUB-BEAM; 60 FIRST SUB-BEAM; 62 SECOND SUB-BEAM; 66, 68, 70, 72, 76 VIRTUAL STRAIGHT LINE; 82 LEFT VENTRICLE; 84 LEFT ATRIUM; PA, PB, PC, PD, PF INTEGRATION START POSITION.

**Claims**

1.  An ultrasound diagnostic apparatus comprising:

    a transmission and reception portion that transmits and receives ultrasound;
    a main beam controller that controls the transmission and reception portion, to scan a main beam formed by ultrasound transmitted and received by the transmission and reception portion;
    a main Doppler measurement portion that Doppler-measures a main beam direction component of a bloodstream velocity for each main beam direction based on ultrasound received by the transmission and reception portion from each main beam direction;
    a calculator that determines an intersecting path direction component of the bloodstream velocity for a position on an intersecting path that intersects each main beam direction based on each main beam direction component of the bloodstream velocity on the intersecting path;
    a sub-beam controller that causes the transmission and reception portion to transmit and receive ultrasound for forming a sub-beam passing through a point on the intersecting path; and
    a sub-Doppler measurement portion that Doppler-measures a sub-beam direction component of the bloodstream velocity at a passing point of the sub-beam on the intersecting path based on ultrasound received by the transmission and reception portion from the sub-beam direction, wherein
    the sub-beam has a direction different from a direction of the main beam passing through the passing point, and
    the calculator determines the intersecting path direction component of the bloodstream velocity using the sub-beam direction component of the bloodstream velocity at the passing point.

2.  The ultrasound diagnostic apparatus according to Claim 1, wherein

    the calculator determines a main beam direction change amount which is an amount of change of each main beam direction component of the bloodstream velocity on the intersecting path in the respective main beam direction, and executes an integration calculation to integrate each main beam direction change amount along the intersecting path, and
    the calculator determines an initial condition of the integration calculation based on an intersecting path direction component of a sub-beam direction component of the bloodstream velocity at the passing point.

3.  The ultrasound diagnostic apparatus according to Claim 2, wherein

    the passing point is a point at one end of the intersecting path, and
    the calculator sets a position of the one end as the initial condition of the integration calculation and executes the integration calculation along the intersecting path.

4.  The ultrasound diagnostic apparatus according to Claim 2, wherein

    the passing point is a partway point on the intersecting path, and
    the calculator comprises:

        a first integrator that sets a position of the partway point as the initial condition and executes the integration calculation along the intersecting path from the partway point on one side;

a second integrator that sets the position of the partway point as the initial condition and executes the integration calculation along the intersecting path from the partway point on the other side; and
a combiner that determines the intersecting path direction component of the bloodstream velocity based on calculation results by the first integrator and the second integrator.

5. The ultrasound diagnostic apparatus according to Claim 2, wherein

the sub-beam controller causes the transmission and reception portion to transmit and receive ultrasound that forms, as the sub-beams, a first sub-beam having one end of the intersecting path as the passing point and a second sub-beam having the other end of the intersecting path as the passing point, and
the calculator comprises:

a first integrator that sets a position of the one end as the initial condition of the integration calculation, and executes the integration calculation along the intersecting path;
a second integrator that sets a position of the other end as the initial condition of the integration calculation, and executes the integration calculation along the intersecting path; and
a combiner that determines the intersecting path direction component of the bloodstream velocity based on calculation results by the first integrator and the second integrator.

6. The ultrasound diagnostic apparatus according to Claim 2, further comprising:

a B-mode controller that controls the transmission and reception portion to scan a B-mode beam formed by ultrasound transmitted and received by the transmission and reception portion;
a tomographic image producer that produces tomographic image data based on ultrasound received by the transmission and reception portion from each B-mode beam direction; and
a velocity calculator that determines the intersecting path direction component of the bloodstream velocity at one end of the intersecting path based on a plurality of tomographic image data produced with elapse of time, wherein
the calculator comprises a first integrator that sets, as the initial conditions, an intersecting path direction component of the bloodstream velocity at the one end and a position of the one end, and executes the integration calculation along the intersecting path,
the passing point is a point at the other end of the intersecting path, and
the calculator further comprises:

a second integrator that sets a position of the other end as the initial condition of the integration calculation, and executes the integration calculation along the intersecting path; and
a combiner that determines the intersecting path direction component of the bloodstream velocity based on calculation results by the first integrator and the second integrator.

7. The ultrasound diagnostic apparatus according to Claim 1, wherein

the sub-beam controller causes the transmission and reception portion to transmit and receive ultrasound for forming an additional sub-beam passing through the passing point,
the sub-Doppler measurement portion determines an additional sub-beam direction component of the bloodstream velocity for the passing point based on ultrasound received by the transmission and reception portion from the additional sub-beam direction,
the additional sub-beam has a direction different from each of directions of the main beam and the sub-beam passing through the passing point, and
the calculator determines the intersecting path direction component of the bloodstream velocity at the passing point using the sub-beam direction component and the additional sub-beam direction component of the bloodstream velocity.

FIG. 1

FIG. 2

FIG. 3

FIG. 4

FIG. 5

FIG. 6

FIG. 7

FIG. 8

42(42SL)    42(42S)    42(42SR)

68

I    G    K

65

J    H    L

β

66

α

42

FIG. 9

42(42SL)    42(42S)    42(42SR)

76

72

65

74

M    G    R

N    U    H

70

42

β

α

FIG. 10

FIG. 11

FIG. 12

FIG. 13

FIG. 14

FIG. 15

**INTERNATIONAL SEARCH REPORT**

| International application No. |
| --- |
| PCT/JP2015/057347 |

| A. CLASSIFICATION OF SUBJECT MATTER |
| --- |
| A61B8/06(2006.01)i, A61B8/14(2006.01)i |

According to International Patent Classification (IPC) or to both national classification and IPC

| B. FIELDS SEARCHED |
| --- |

Minimum documentation searched (classification system followed by classification symbols)
A61B8/00-8/15, G01S15/89, G10K11/34

Documentation searched other than minimum documentation to the extent that such documents are included in the fields searched
Jitsuyo Shinan Koho          1922-1996   Jitsuyo Shinan Toroku Koho   1996-2015
Kokai Jitsuyo Shinan Koho    1971-2015   Toroku Jitsuyo Shinan Koho   1994-2015

Electronic data base consulted during the international search (name of data base and, where practicable, search terms used)
JSTPlus/JMEDPlus/JST7580(JDreamIII), CiNii

| C. DOCUMENTS CONSIDERED TO BE RELEVANT |
| --- |

| Category* | Citation of document, with indication, where appropriate, of the relevant passages | Relevant to claim No. |
| --- | --- | --- |
| A | JP 2013-192643 A  (The University of Tokyo), 30 September 2013 (30.09.2013), entire text; all drawings | 1-7 |
| A | JP 2013-165922 A  (Hitachi Aloka Medical, Ltd.), 29 August 2013 (29.08.2013), abstract | 1-7 |
| A | JP 2001-258887 A  (GE Medical Systems Global Technology Company, L.L.C.), 25 September 2001 (25.09.2001), paragraphs [0055], [0089]; fig. 5 | 1-7 |
| A | JP 2002-17726 A  (Shigeo OTSUKI), 22 January 2002 (22.01.2002), abstract | 1-7 |

| ☒ | Further documents are listed in the continuation of Box C. | ☒ | See patent family annex. |
| --- | --- | --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 27 March 2015 (27.03.15) | 07 April 2015 (07.04.15) |

| Name and mailing address of the ISA/ Japan Patent Office 3-4-3,Kasumigaseki,Chiyoda-ku, Tokyo 100-8915,Japan | Authorized officer Telephone No. |
| --- | --- |

Form PCT/ISA/210 (second sheet) (July 2009)

**INTERNATIONAL SEARCH REPORT**

International application No.

PCT/JP2015/057347

C (Continuation).   DOCUMENTS CONSIDERED TO BE RELEVANT

| Category* | Citation of document, with indication, where appropriate, of the relevant passages | Relevant to claim No. |
|---|---|---|
| A | Kageyoshi KATAKURA et al., "Method for ultrasonic vector velocity measurement", Journal of the Acoustical Society of Japan, 25 December 1995 (25.12.1995), vol.52, no.1, pages 10 to 18 | 1-7 |
| A | JP 56-119237 A  (Tokyo Shibaura Electric Co., Ltd.), 18 September 1981 (18.09.1981), claims; fig. 2 | 1-7 |

Form PCT/ISA/210 (continuation of second sheet) (July 2009)

**INTERNATIONAL SEARCH REPORT**
Information on patent family members

| International application No. |
| --- |
| PCT/JP2015/057347 |

| | | | |
| --- | --- | --- | --- |
| JP 2013-192643 A | 2013.09.30 | JP 5497821 B2 | 2014.05.21 |
| | | CN 104168835 A | 2014.11.26 |
| | | EP 2826425 A1 | 2015.01.21 |
| | | US 2015/013471 A1 | 2015.01.15 |
| | | WO 2013/136573 A1 | 2013.09.19 |
| JP 2013-165922 A | 2013.08.29 | (Family: none) | |
| JP 2001-258887 A | 2001.09.25 | JP 4716346 B2 | 2011.07.06 |
| | | CN 1332360 A | 2002.01.23 |
| | | CN 1222757 C | 2005.10.12 |
| | | EP 1121901 A2 | 2001.08.08 |
| | | EP 1121901 A9 | 2002.06.12 |
| | | KR 2001-0087166 A | 2001.09.15 |
| | | KR 100737029 B1 | 2007.07.09 |
| | | US 6535835 B1 | 2003.03.18 |
| JP 2002-017726 A | 2002.01.22 | (Family: none) | |
| JP 56-119237 A | 1981.09.18 | JP 627856 B2 | 1987.02.19 |
| | | AU 530259 B | 1983.07.07 |
| | | AU 6746681 A | 1981.09.03 |
| | | DE 3174830 D1 | 1986.07.24 |
| | | EP 35213 A1 | 1981.09.09 |
| | | EP 35213 B1 | 1986.06.18 |
| | | PH 18098 A | 1985.03.20 |
| | | PH 18148 A | 1985.04.03 |
| | | US 4373533 A | 1983.02.15 |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013192643 A
- JP 2013165922 A